Europäisches Patentamt

European Patent Office

Office européen des brevets

(11) Veröffentlichungsnummer: **0 490 137 A2**

# EUROPÄISCHE PATENTANMELDUNG

(21) Anmeldenummer: **91119861.2**

(22) Anmeldetag: **21.11.91**

(51) Int. Cl.5: **A41D 13/00**, A63B 71/08, A42B 3/12, A43B 7/32, A61F 5/04

(30) Priorität: **10.12.90 DE 9016713 U**

(43) Veröffentlichungstag der Anmeldung: **17.06.92 Patentblatt 92/25**

(84) Benannte Vertragsstaaten: **DE IT SE**

(71) Anmelder: **W.L. Gore & Associates GmbH**
**Hermann-Oberth-Strasse 22**
**W-8011 Putzbrunn(DE)**

(72) Erfinder: **Siegl, Klaus**
**Rosenheimerstrasse 44a**
**W-8018 Grafing(DE)**

(74) Vertreter: **Klunker . Schmitt-Nilson . Hirsch**
**Winzererstrasse 106**
**W-8000 München 40(DE)**

(54) **Körperschutzvorrichtung.**

(57) Körperschutzvorrichtung, beispielsweise zur Anordnung an oder in einem Bekleidungsstück, mit einer Hartmaterialschale und einem Polsterteil aus geschlossenporigem Schaumstoff, das auf der dem menschlichen Körper zugewandten Seite der Hartmaterialschale angeordnet ist, wobei sowohl die Hartmaterialschale als auch das Polsterteil quer zu ihrer Längserstreckungsrichtung gelocht sind.

Schnitt A — B

Aussen————————————Innen

FIG. 3

Die Erfindung betrifft eine Körperschutzvorrichtung, mit der bestimmte Körperteile gegen Aufprall, Schlageinwirkung oder ähnliches geschützt werden können. Solche Körperschutzvorrichtungen werden in bekannten Eishockeyhosen verwendet, wo sie als Nieren-, Gesäß-, oder Oberschenkelschutz dienen. Für diesen Verwendungszweck sind sie an ihrer Zweckbestimmung entsprechender Stelle an der Eishockeyhose festgenäht oder in für sie vorgesehene Taschen der Eishockeyhose eingenäht.

Mitunter werden solche Körperschutzvorrichtungen auch separat von der Eishockeyhose angezogen.

Für solche Körperschutzvorrichtungen bekannter Art gibt es unterschiedliche Aufbauten. Diesen Aufbauten ist gemeinsam, daß sie eine Hartmaterialschale aufweisen, deren Form in etwa derjenigen der speziellen Körperschutzvorrichtung entspricht. Dabei kann die Hartmaterialschale z. B. aus Kunststoff, Metall oder aus einem festen gehärteten Kunststoffschaum bestehen. Gemeinsam ist den bekannten Lösungen, daß beidseits der Hartmaterialschale mindestens ein Polsterteil vorgesehen ist. Dieses besteht bei einer bekannten Lösung aus geschlossenporigem Schaum, bei einer anderen bekannten Lösung aus offenporigem Schaum. Bei der einen bekannten Lösung ist auf der zum Körper des Trägers der Eishockeyhose weisenden Seite der Hartmaterialschale zusätzlich oder anstelle des Polsterteils aus Schaumstoff eine blasenförmige Lufteinschlüsse aufweisende Folie angeordnet, wie sie auch als druckaufnehmende Verpackungsfolie bekannt ist.

Auf der Innenseite einer solchen Körperschutzvorrichtung befindet sich üblicherweise ein Futter. Über deren Außenseite erstreckt sich das Außenmaterial der Eishockeyhose, bei dem es sich beispielsweise um ein Polyamid-Gewebe oder um polyesterbeschichtetes Textilmaterial handelt.

Die Hartmaterialschalen sollen Aufprall- oder Aufschlagkräfte abfangen. Die inneren Polsterteile dämpfen einerseits den bei Schlageinwirkung auf das Kunststoffteil vom Kunststoffteil auf den Körper des Benutzers der Eishockeyhose erfolgenden Aufprall und erhöhen andererseits den Tragekomfort einer mit solchen Körperschutzvorrichtungen ausgerüsteten Eishockeyhose. Die auf der Außenseite der Hartmaterialschale befindlichen Polsterteile dämpfen einerseits auf solche Körperschutzvorrichtungen einwirkende Schläge und schützen andererseits andere Eishockeyspieler, die mit dem Träger einer solchen Eishockeyhose zusammenprallen, vor einem harten Aufschlagen auf die Hartmaterialschale der Körperschutzvorrichtung.

Sportarten wie Eishockey führen zu einer starken Erhitzung und entsprechend starkem Schwitzen der Spieler. Um den Spielern einen möglichst guten Prall- und Schlagschutz zu bieten, ist ein großer Teil der Fläche einer Eishockeyhose mit Körperschutzvorrichtungen versehen. Da die Hartmaterialschalen der Körperschutzvorrichtungen sowohl wasserundurchlässig als wasserdampfundurchlässig sind, kommt es unter den Körperschutzvorrichtungen zu sehr starker Schweißansammlung und eventuell zu einem großen Hitzestau.

Wird als Polstermaterial auf der Innenseite der Hartmaterialschale offenporiger Schaumstoff verwendet, saugt dieser sich mit dem Schweiß des Eishockeyspielers voll wie ein Schwamm. Da Eishockeyspieler während eines Spiels mehrere Liter Wasser ausschwitzen können, kann die in dem offenporigen Schaumstoff festgehaltene Schweißmenge enorm sein. Dies führt nicht nur zu einer starken Beeinträchtigung des Tragekomforts einer solchen Eishockeyhose, sondern macht eine solche Eishockeyhose auch entsprechend schwer.

Das Vollsaugen mit Schweiß wird bei Körperschutzvorrichtungen verhindert, deren Polsterteile aus geschlossenporigem Schaumstoff bestehen. Allerdings führt dies dazu, daß nichts von dem beim Spielen erzeugten Schweiß von der Körperoberfläche des Spielers abgenommen wird, sondern der gesamte Schweiß am Körper des Spielers verbleibt, soweit er nicht innerhalb der Körperschutzvorrichtungen der Eishockeyhose am Körper des Spielers herabläuft.

Beide bekannten Körperschutzvorrichtungen führen somit zu einem wenig angenehmen Trageverhalten der damit ausgerüsteten Eishockeyhosen.

Der Erfindung liegt die Aufgabe zugrunde, Körperschutzvorrichtungen mit wesentlich erhöhtem Tragekomfort verfügbar zu machen.

Die Lösung dieser Aufgabe besteht in einer Körperschutzvorrichtung mit einer Hartmaterialschale und einem die Hartmaterialschale auf der dem menschlichen Körper zugewandten Seite abdeckenden Polsterteil aus geschlossenporigem Schaumstoff, wobei sowohl die Hartmaterialschale als auch das Polsterteil quer zu ihrer Längserstreckungsrichtung gelocht sind. Vorzugsweise fluchten die Lochungen der Hartmaterialschale und des Polsterteils mindestens für den Fall, daß Hartmaterialschale und Polsterteil fest miteinander verbunden sind.

Wird beidseits der Hartmaterialschale ein Polsterteil verwendet, sind beide Polsterteile mit Querlochung versehen.

Die erfindungsgemäße Querlochung führt zu dem Vorteil, daß die Körperschutzvorrichtung wasserdampfdurchlässig ist, so daß durch Schwitzen entstehender Wasserdampf durch die Körperschutzvorrichtung hindurch entweichen kann und daher die Schweißansammlung am Körper des Trägers der Körperschutzvorrichtung vermieden oder mindestens stark verringert wird. Da für das Polsterteil beziehungsweise die Polsterteile geschlossenporiger Schaumstoff verwendet wird, kann sich der Schaumstoff nicht mit Schweißflüssigkeit vollsaugen.

Am Körper oder innerhalb der Körperschutzvorrichtung kondensierter Schweiß kann über die Löcher der Körperschutzvorrichtung vom Körper weg ablaufen.

Wird die erfindungsgemäße Körperschutzvorrichtung in einem Bekleidungsstück wie einer Eishockeyhose benutzt, verwendet man für das auf der Außenseite der Körperschutzvorrichtung befindliche Material des Bekleidungsstückes vorzugsweise wasserdichtes, wasserdampfdurchlässiges Material, beispielsweise in Form eines Laminates, das auf der Außenseite ein Textilgewebe und auf dessen Innenseite eine Funktionsschicht aus wasserdichtem, wasserdampfdurchlässigem Material aufweist. Wird das Bekleidungsstück im Regen getragen oder, wie im Fall einer Eishockeyhose, Schmelzwasser auf der Eisfläche ausgesetzt, kann einerseits das Wasser nicht bis zum Körper des Trägers des Bekleidungsstückes Vordringen, kann aber andererseits beim Schwitzen entstehender Wasserdampf des Trägers durch die Querlochungen der Körperschutzvorrichtung und durch das wasserdampfdurchlässige Außenmaterial entweichen.

Die Einsatzmöglichkeiten der erfindungsgemäßen Körperschutzvorrichtung sind vielfältig. Einerseits können sie als Einsätze oder Einlagen in Bekleidungsstücken, insbesondere Sportbekleidungsstücken verwendet werden. Beispiele hierfür sind Hosen und Trikots für Sportarten wie Eishockey, Fußball, American Football, Rugby, Handball usw. Andererseits können sie auch unabhängig von Kleidungsstücken verwendete werden. Ein Beispiel sind Schienbeinschoner für Fußballer, die vom Fußballspieler vor dem Spiel zwischen Schienbein und Strumpf eingeschoben werden können.

Es gibt zahlreiche weitere Anwendungen für erfindungsgemäße Körperschutzvorrichtungen. Beispiele sind Schutzhelme, die entweder mit solchen Körperschutzvorrichtungen versehen sind oder insgesamt durch eine entsprechend geformte Körperschutzvorrichtung gebildet sind.

Ein weiteres Beispiel sind Sicherheitsschuhe, die mindestens im Zehenbereich mit einer steifen Schutzkappe Versehen sind.

Die erfindungsgemäße Körperschutzvorrichtung eignet sich auch für Körperschienungszwecke, beispielsweise als Ersatz für Gipsschalen, mittels welchen Knochenbrüche im Arm- oder Beinbereich geschient werden.

Bei den meisten Verwendungsarten erfindungsgemäßer Körperschutzvorrichtungen ist es von Vorteil, auf der Außenseite der Körperschutzvorrichtung wasserdampfdurchlässiges Material zu verwenden, das außerdem winddicht und/oder wasserdicht ist. Dies gilt nicht nur für Bekleidungsgegenstände, Schutzhelme und Sicherheitsschuhe, sondern auch für Körperschienungsvorrichtungen. Denn einerseits muß das Außenmaterial wasserdampfdurchläddig sein, um die Wirkung der Querlochung der Körperschutzvorrichtung nicht zu unterbinden. Andererseits ist durch die Querlochung ein leichtes Durchdringen der Körperschutzvorrichtung mit Wasser ermöglicht, was durch wasserdichtes Außenmaterial verhindert werden kann. Zu diesem Zweck versieht man das Außenmaterial mit einer wasserdichten, wasserdampfdurchlässigen Funktionsschicht bekannter Art, vorzugsweise in Form eines Laminates mit einem wasserdurchlässigen Außenmaterial und der Funktionsschicht.

Das mikroporöse polymere Material kann mittels irgendeines bekannten Verfahrens aus irgendeinem polymeren Material hergestellt werden, das sich zur Formung einer Funktionsschichtfolie eignet. Zur Formung einer mikroporösen polymeren Matrix geeignete Polymere umfassen Polyolefine, wie Polyethylen-Polypropylen-copolymere, Polyethylen-Terephthalate, Polycaprolactam, Polyvinylidenfluorid, Polybutylenterephthalat, Polyestercopolymere und Polytetrafluorethylen.

Die Erfindung wird nun anhand von Ausführungsformen näher erläutert. In den Zeichnungen zeigen:

Fig. 1  eine schematische Darstellung einer Eishockeyhose mit mehreren Körperschutzvorrichtungen;

Fig. 2  eine ausschnittsweise Querschnittsdarstellung einer Eishockeyhose mit einer herkömmlichen Körperschutzvorrichtung;

Fig. 3  eine Querschnittsdarstellung einer Eishockeyhose mit einer erfindungsgemäßen Körperschutzvorrichtung;

Fig. 4  eine schematische Querschnittsdarstellung eines erfindungsgemäß ausgebildeten Schutzhelms;

Fig. 5  eine schematische Querschnittsdarstellung eines erfindungsgemäß ausgestalteten Sicherheitsschuhs; und

Fig. 6  eine Querschnittsdarstellung einer erfindungsgemäß ausgebildeten Körperschienungsvorrichtung.

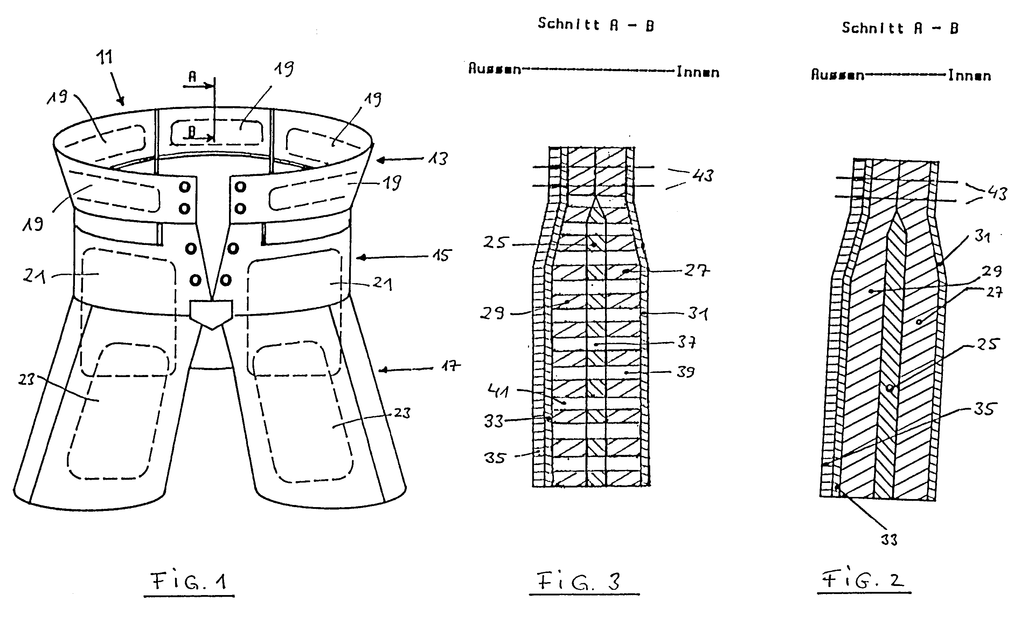

Fig. 1 zeigt in schematisierter Darstellung eine Eishockeyhose 11 mit einem Bundbereich 13, einem Hüftbereich 15 und einem Oberschenkelbereich 17. Im Bundbereich 13 sind Bund- und Nierenschutzvorrichtungen 19 angeordnet. Im Hüftbereich 15 befinden sich Hüftschutzvorrichtungen 21. Im Oberschenkelbereich 17 ist die Eishockeyhose 11 mit Oberschenkelschutzvorrichtungen 23 versehen.

Fig. 2 zeigt in Querschnittsdarstellung den Aufbau einer bekannten Körperschutzvorrichtung für eine Eishockeyhose 11. Den Kern der Körperschutzvorrichtung bildet eine plattenförmige Hartmaterialschale 25, gegebenenfalls mit einer an die Körperform anpassenden Krümmung. Die Hartmaterialschale 25 besteht aus einem Hartkunststoff, wie er beispielsweise für die Herstellung von Schuhlöffeln verwendet wird. Die Hartmaterialschale 25 dient als Schlag und Prallschutz.

Beidseits der Hartmaterialschale 25 ist je ein Polsterteil angeordnet, nämlich ein zum Körper weisendes inneres Polsterteil 27 und ein auf der anderen Seite der Hartmaterialschale 25 befindliches äußeres Polsterteil 29. Beide Polsterteile 27 und 29 bestehen aus geschlossenporigem Schaumstoff.

Auf der Innenseite des inneren Polsterteils 27 befindet sich ein Innenfutter 31. Auf der äußeren Seite des äußeren Polsterteils 29 befindet sich ein Außenfutter 33. Über dem Außerfutter 33 verläuft Außenmaterial 35, das durch ein Polyamid-Gewebe gebildet ist.

Fig. 2 zeigt zwar eine Querschnittsansicht einer Bund- und und Nierenschutzvorrichtung 19 längs der Schnittlinie A-B in Fig. 1. Diese Darstellung repräsentiert aber gleichzeitig den Aufbau der weiteren Körperschutzvorrichtungen, nämlich der Hüftschutzvorrichtungen 21 und der Oberschenkelschutzvorrichtungen 23.

Fig. 3 zeigt eine Fig. 2 entsprechende Querschnittsdarstellung einer erfindudngsgemäßen Bund- und Nierenschutzvorrichtung 19. Wie die bekannte Körperschutzvorrichtung weist auch die erfindungsgemäße Körperschutzvorrichtung eine Hartmaterialschale 25, ein inneres Polsterteil 27 und ein äußeres Polsterteil 29 auf. Erfindungsgemäß sind allerdings die Hartmaterialschale 25 und die beiden Polsterteile 27 und 29 je mit quer zu deren Längserstreckungsrichtung verlaufenden Löchern 37 bzw. 39 bzw. 41 vorgesehen. Diese weisen vorzugsweise einen relativ großen Lochdurchmesser auf, damit eine gute Wasserdampfdurchlässigkeit erreicht wird. Beispielsweise beträgt der Lochdurchmesser 4 mm.

Auch bei der erfindungsgemäßen Körperschutzvorrichtung ist die Innenseite des inneren Polsterteils 27 von einem Innenfutter 31 bedeckt, während auf der Außenseite des äußeren Polsterteils 29 ein Außenfutter 33 angeordnet ist. Ein Außenmaterial 35 der erfindungsgemäßen Körperschutzvorrichtung besteht vorzugsweise aus einem Laminat, das ein textiles Gewebe als Außenmaterial und eine auf dessen Innenseite befindliche wasserdichte, wasserdampfdurchlässige und winddichte Funktionsschicht aufweist.

Aufgrund der Wasserdichtigkeit des Außenmaterials 35 kann kein Wasser von außen durch die Körperschutzvorrichtung hindurch zum Körper des Benutzers der Eishockeyhose 11 gelangen. Das Außenmaterial ist jedoch durchlässig für Wasserdampf, der durch das Innenfutter 31, die Löcher 37, 39 und 41 und das Außenfutter 33 hindurchgelangt.

Die Hartmaterialschale 25, die Polsterteile 27, 29, das Innenfutter 31, das Außenfutter 33 und das Außenmaterial 35 sind vorzugsweise nur lose aneinandergelegt und lediglich in ihrem in Fig. 3 oberen Bereich mittels zweier paralleler Nähte 43 aneinander befestigt. Es besteht aber auch die Möglichkeit, mindestens die Hartmaterialschale 25 und die beiden Polsterteile 27 und 29 miteinander zu verkleben. Besonders im letzteren Fall empfiehlt es sich, die Löcher 27, 39 und 41 miteinander fluchtend auszubilden, damit eine gute Wasserdampfdurchlässigkeit erreicht wird. Vorzugsweise werden die Hartmaterialschale 25 und die beiden Polsterteile 27 und 29 aneinanderliegend gelocht, um ein gutes Fluchten der Löcher 37, 39 und 41 zu erzielen. Werden die Hartmaterialschale 25 und die beiden Polsterteile 27 und 29 nicht miteinander verklebt, sondern nur lose aneinandergelegt, ist ein Fluchten der Löcher 37, 39 und 41 nicht unbedingt erforderlich, da dann infolge von Bewegungen des Trägers der Eishockeyhose 11 Quer-Relativbewegungen zwischen der Hartmaterialschale 21 und den beiden Polsterteilen 27 und 29 entstehen, in deren Folge zwischen diesen drei Teilen Luftspalte entstehen, über welche auch nicht-fluchtende Löcher 37, 39 und 41 miteinander verbunden werden.

Die in Fig. 3 gezeigte Körperschutzvorrichtung kann entweder mittels der Nähte 43 an der Eishokkeyhose 11 befestigt werden. Eine andere Möglichkeit besteht darin, die Körperschutzvorrichtungen in Taschen unterzubringen, die zwischen dem Außenmaterial 35 und dem Außenfutter 33 einerseits und dem Innenfutter 31 andererseits gebildet werden. Die Körperschutzvorrichtungen können aber auch mittels einer separaten Vorrichtung getragen werden.

Nachdem anhand der Fig. 1 bis 3 eine spezielle Verwendungsart von hier beschriebenen Körperschutzvorrichtungen erläutert worden ist, folgen weitere Anwendungen, die in den Fig. 4 bis 6 dargestellt sind.

Dabei werden zur Vereinfachung für Schichten und Materialien, die mit Schichten beziehungsweise mit Materialien der Fig. 3 übereinstimmen, gleiche Bezugszeichen verwendet. Nicht gezeigt sind in den Fig. 4 bis 6 die Querlöcher in den Hartmaterialschalen und den Polsterteilen. Erfindungsgemäß sind diese aber dennoch mit Löchern versehen, entsprechend den Löchern 37, 39 und 41 in Fig. 3.

Fig. 4 zeigt eine schematische Querschnittsdarstellung eines Schutzhelms. Dieser ist durch eine einzige Körperschutzvorrichtung gebildet, die in Helmform gebracht worden ist. Kernstück und Prall- sowie Schlagschutz bildet die Hartmaterialschale 25. Diese ist in ein Polsterteil eingebettet, das sowohl das innere Polsterteil 27 als auch das äußere Polsterteil 29 bildet und um den Rand 45 der Hartmaterialschale herumgeführt ist, um den Rand 45 abzupolstern. Die Innenseite des inneren Polsterteils 27 ist mit dem Innenfutter 31 ausgekleidet. Die Außenseite des äußeren Polsterteils 29 ist mit Außenmaterial 35 überzogen, das um den Rand 45 der Hartmaterialschale 25 herumgeführt und mittels einer Naht 47 mit dem Futter 31 vernäht ist, wobei die Naht im inneren Randbereich des inneren Polsterteils 27 verläuft.

Da die Hartmaterialschale 25 und die beiden Polsterteile 27 und 29 mit in Fig. 4 nicht gezeigten Querlöchern versehen sind, besteht zunächst Wasserdurchlässigkeit des Schutzhelms. Um den Schutzhelm wasserdicht zu machen und dennoch die mittels der Löcher erreichte Wasserdampfdurchlässigkeit aufrechtzuerhalten, weist das Außenmaterial 35 vorzugsweise eine wasserdichte, wasserdampfdurchlässige Funktionsschicht auf.

Es ist nicht unbedingt erforderlich, die Hartmaterialschale 25 gänzlich in Polstermaterial einzubetten. Man kann das äußere Polsterteil 29 auch weglassen und das Außenmaterial 35 direkt auf der Hartmaterialschale 25 anordnen.

In vielen Fällen wird es erwünscht sein, daß das Außenmaterial des Schutzhelms aus Hartmaterial besteht, z. B. aus Kunststoff. Hierfür kann man dann in Abweichung von der in Fig. 4 dargestellten Ausführungsform die Hartmaterialschale 25 als Außenmaterial nehmen und gegebenenfalls eine wasser- und winddichte sowie wasserdampfdurchlässige Funktionsschicht innerhalb der Hartmaterialschale 25 anordnen.

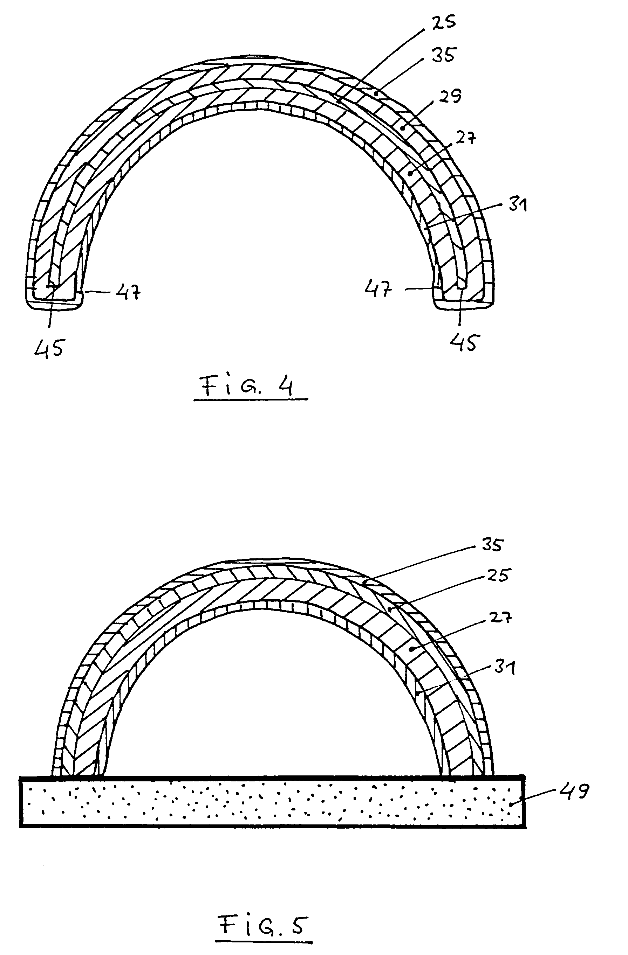

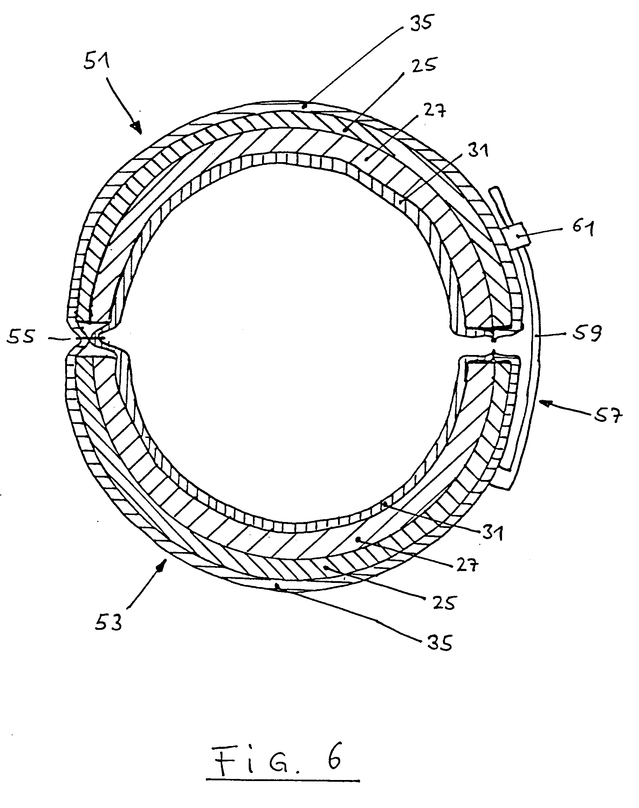

Fig. 5 zeigt einen schematischen Aufbau eines Sicherheitsschuhs, der einen Schlag- und Prallschutz in Form einer Hartmaterialschale 25 aufweist, und zwar mindestens im Zehenbereich des Schuhs. Bei der in Fig. 5 gezeigten Ausführungsform befindet sich auf der Innenseite der Hartmaterialschale 25 ein inneres Polsterteil 27. Es kann aber auch zusätzlich ein äußeres Polsterteil 29 auf der Außenseite der Hartmaterialschale 25 angeordnet werden. Die Hartmaterialschale 25 und das innere Polsterteil 27 und gegebenenfalls auch ein äußeres Polsterteil sind wieder in Querrichtung gelocht, obwohl dies in Fig. 6 nicht dargestellt ist. Die Innenseite des inneren Polsterteils 27 ist mit einem Futter 31 ausgekleidet und die Außenseite der Hartmaterialschale 25 ist mit einem Außenmaterial 35 überzogen. Bei dem Außenmaterial 35 handelt es sich um das Schaftmaterial des Sicherheitsschuhs. Soll dieser wasserdicht sein, enthält das Außenmaterial 35 eine wasserdichte, wasserdampfdurchlässige Funktionsschicht, um die mit den Löchern der Hartmaterialschale 25 und des inneren Polsterteils 27 erreichte Wasserdampfdurchlässigkeit auch für das Außenmaterial zu schaffen.

Man kann die Funktionsschicht auch innerhalb der Hartmaterialschale 25 anordnen, um ihr einen besseren mechanischen Schutz zu verschaffen.

An den unteren Rand von Außenmaterial 35, Hartmaterialschale 25, Polsterteil 27 und Innenfutter 31 ist eine Laufsohle 49 angespritzt. Es kann aber auch eine beliebige andere Laufsohle verwendet werden.

Fig. 6 zeigt in schematischer Querschnittsdarstellung eine röhrenförmige Körperschienungsvorrichtung, beispielsweise zur Schienung eines gebrochenen Armes oder eines gebrochenen Beines. Bei der in Fig. 6 gezeigten Ausführungsform besteht die Körperschienungsvorrichtung aus zwei Schienungshalbschalen, nämlich einer in Fig. 6 oberen Schienungshalbschale 51 und einer in Fig. 6 unteren Schienungshalbschale 53. Jede der beiden Schienungshalbschalen 51 und 53 weist eine im wesentlichen halbkreisförmige Hartmaterialschale 25 und auf deren Innenseite ein inneres Polsterteil 27 auf, die beide mit in Fig. 6 nicht gezeigten, querverlaufenden Lochungen versehen sind.

Auf der Innenseite eines jeden inneren Polsterteils 27 befindet sich ein Innenfutter 31, um den Tragekomfort der Körperschienungsvorrichtung zu erhöhen. Jede der beiden Hartmaterialschalen 25 können mit einem Außenmaterial 35 überzogen sein. Dieses kann eine wasserdichte, wasserdampfdurchlässige Funktionsschicht aufweisen, um das Eindringen von Wasser durch die Löcher der Hartmaterialschalen 25 und des inneren Polsterteils 27 hindurch zu verhindern. Die Funktionsschicht kann auch innerhalb der Hartmaterialschalen 25 angeordnet sein. Bei Ausrüstung mit einer solchen Funktionsschicht kann diese Körperschienungsvorrichtung problemlos auch im Freien getragen werden, selbst wenn es regnet oder schneit. Auch gibt dies die Möglichkeit, die Körperschienungsvorrichtung beim Duschen anzubehalten. Die Notwendigkeit, vor dem Duschen die Körperschienungsvorrichtung mit einer Plastikfolie oder einer Plastiktüte abzudecken, wie es häufig beim Tragen einer Gipsschienungsschale getan wird, entfällt damit.

Da die beiden Hartmaterialschalen 25 üblicherweise aus einem steifen Kunststoffmaterial gebildet sind, ist die in Fig. 6 gezeigte Körperschienungsvorrichtung aufklappbar ausgebildet. Zu diesem Zweck erstrecken sich das Innenfutter 31 und das Außenmaterial 35 über beide Hartmaterialschalen 25, wobei sie zwischen den beiden Hartmaterialschalenrändern auf der in Fig. 6 linken Seite mittels beispielsweise einer Naht 55 aneinander befestigt sind. An den in Fig. 6 rechten Rändern der Schienungshalbschalen 51 und 53 sind das Innenfutter 31 und das Außenmaterial 35 je miteinander verbunden, ohne zu dem Innenfutter 31 bzw. dem Außenmaterial 35 der gegenüberliegenden Schienungshalbschale 53 hinüberzureichen. Auf diese Weise können die beiden Schienungshalbschalen 51 und 53 relativ zueinander bewegt werden, um sie in eine Offenstellung oder in eine Schließstellung zu bewegen, wobei der mit der Naht 53 verbundene Bereich von Innenfutter 31 und Außenmaterial 35 ein Schwenkscharnier bildet. Die beiden nicht miteinander verbundenen Ränder der beiden Schienungshalbschalen 51 und 53 können mit Hilfe einer Verschlußvorrichtung 57 aneinander gehalten werden, die einen Verschlußbügel 59 aufweist, der an der unteren Schienungshalbschale 53 verankert ist und durch eine an der oberen Schienungshalbschale 51 angebrachte Verschlußöse 61 hindurchgeführt ist, innerhalb welcher er in beliebigen Stellungen verrastbar ist. Hierfür kann ein bekannter Verrastmechanismus gewählt werden, so daß dessen genaue Beschreibung nicht erforderlich ist.

Zum Anlegen dieser Körperschienungsvorrichtung werden die die beiden Schienungshalbschalen 51 und 53 in Offengeschwenkt, unter Lösen der Verriegelung zwischen dem Verschlußbügel 59 und der Verschlußöse 61.

Nach Einlegen des zu schienenden Körperteils, beispielsweise eines gebrochenen Armes, in die eine Schienungshalbschale werden die beiden Schienungshalbschalen 51 und 53 in Schließstellung gebracht und mit Hilfe der Verschlußvorrichtung 57 in Schließstellung gehalten. Ist der Verschlußbügel 59 genügend lang, kann die Körperschienungsvorrichtung an unterschiedliche dicke Arme und Beine angepaßt werden, indem die beiden Schienungshalbschalen 51 und 53 mehr oder weniger dicht in Schließstellung gebracht werden.

Wenn absolute Wasserdichtigkeit erwünscht ist, kann der zwischen den beiden Schienungshalbschalen 51 und 53 auf Verschlußseite verbleibende Spalt mit wasserdichtem Material abgedeckt werden. Eine Möglichkeit hierfür besteht darin, über die an den zu schienenden Körperteil angelegten Schienungshalbschalen 51 und 53 einen Schlauch aus einem elastischen, eine wasserdichte, wasserdampfdurchlässige Funktionsschicht enthaltendem Laminat zu ziehen, der gleichzeitig die Verschlußvorrichtung 57 und auch das Außenmaterial 35 ersetzen kann.

**Patentansprüche**

1. Körperschutzvorrichtung, beispielsweise zur Anordnung an oder in einem Bekleidungsstück, mit einer Hartmaterialschale (25) und einem Polsterteil (27) aus geschlossenporigem Schaumstoff, das auf der dem menschlichen Körper zugewandten Seite der Hartmaterialschale (25) angeordnet ist, dadurch gekennzeichnet, daß sowohl die Hartmaterialschale (25) als auch das Polsterteil (27) quer zu ihrer Längserstreckungsrichtung gelocht sind (37, 39).

2. Körperschutzvorrichtung nach Anspruch 1, dadurch gekennzeichnet, daß das Polsterteil (27) auf der dem menschlichen Körper zugewandten Seite mit einem wasserdampfdurchlässigen Futter (31) versehen ist.

3. Körperschutzvorrichtung nach Anspruch 1 oder 2, dadurch gekennzeichnet, daß beidseits der Hartmaterialschale (25) je ein quergelochtes Polstersteil (27, 29) aus geschlossenporigem Schaumstoff angeordnet ist.

4. Körperschutzvorrichtung nach einem der Ansprüche 1 bis 3, dadurch gekennzeichnet, daß mindestens dann, wenn die Hartmaterialschale (25) und das Polsterteil (27) bzw. die Polsterteile (27, 29) fest miteinander verbunden sind, die Löcher (37, 39, 41) der Hartmaterialschale (25) und des Polsterteils (27) bzw. der Polsterteile (27, 29) im wesentlichen miteinander fluchten.

5. Verwendung der Körperschutzvorrichtung nach einem der Ansprüche 1 bis 4 als Schutzeinlage in einem Sportbekleidungsstück, insbesondere für Sportarten wie Hockey, Eishockey, American Football, Ragby und Handball.

6. Verwendung der Körperschutzvorrichtung nach einem der Ansprüche 1 bis 4 für einen Schutzhelm, insbesondere für Motorradfahrer, Radfahrer, Skifahrer, Handwerker, Feuerwehrleute und Soldaten.

7. Verwendung der Körperschutzvorrichtung nach einem der Ansprüche 1 bis 4 als Schienungsvorrichtung, z.B. als Ersatz für Gipsschalen oder Gipsschienen.

8. Bekleidungsstück mit mindestens einer daran angeordneten oder darin eingesetzten Körperschutzvorrichtung und einer Hartmaterialschale (25) und einem Polsterteil (27) aus geschlossenporigem Schaumstoff, das auf der dem menschlichen Körper zugewandten Seite der Hartmaterialschale (25) angeordnet ist, dadurch gekennzeichnet, daß sowohl die Hartmaterialschale (25) als auch das Polsterteil (27) quer zu ihrer Längserstreckungsrichtung gelocht sind.

9. Bekleidungsstück nach Anspruch 8, dadurch gekennzeichnet, daß die Körperschutzvorrichtung zwischen einem Außenmaterial (35) und einem Innenfutter (31) des Bekleidungsstückes angeordnet ist.

10. Bekleidungsstück nach Anspruch 8, dadurch gekennzeichnet, daß die Körperschutzvorrichtung auf der Innenseite des Materials des Bekleidungsstückes angeordnet ist.

11. Bekleidungsstück nach einem der Ansprüche 8 bis 10, dadurch gekennzeichnet, daß das Außenmaterial (35) bzw. das Material des Bekleidungsstücks eine wasserdampfdurchlässige sowie winddichte und/oder wasserdichte Funktionsschicht aufweist.

12. Bekleidungsstück nach Anspruch 11, dadurch gekennzeichnet, daß das Außenmaterial (35) bzw. Material des Bekleidungsstücks ein die Funktionsschicht aufweisendes Laminat aufweist.

13. Bekleidungsstück nach Anspruch 11 oder 12, dadurch gekennzeichnet, daß die Funktionsschicht eine Membran aus gerecktem, mikroporösem Polytetrafluorethylen (PTFE) aufweist.

14. Bekleidungsstück nach einem der Ansprüche 8 bis 13, dadurch gekennzeichnet, daß im Nieren-, Gesäß-, Hüft- und Oberschenkelbereich (13, 15, 17) je mindestens eine Körperschutzvorrichtung angeordnet ist.

15. Schutzhelm mit einer Hartmaterialschale (25), dadurch gekennzeichnet, daß die Hartmaterialschale (25) auf ihrer Innenseite mit mindestens einem Polsterteil (27) aus geschlossenporigem Schaumstoff versehen ist und daß sowohl die Hartmaterialschale (25) als auch das Polsterteil (27) quer zu ihrer Längserstreckungsrichtung gelocht sind.

16. Schutzhelm nach Anspruch 15, dadurch gekennzeichnet, daß das Polsterteil (27) auf seiner Innenseite mit einem wasserdampfdurchlässigen Futter versehen ist.

17. Schutzhelm nach Anspruch 15 oder 16, dadurch gekennzeichnet, daß außerhalb oder innerhalb der Hartmaterialschale (25) ein Material (35) angeordnet ist, das eine wasserdampfdurchlässige sowie winddichte und/oder wasserdichte Funktionsschicht aufweist.

18. Schutzhelm nach Anspruch 17, dadurch gekennzeichnet, daß das Material (35) ein die Funktionsschicht enthaltendes Laminat aufweist.

19. Schutzhelm nach Anspruch 17 oder 18, dadurch gekennzeichnet, daß die Funktionsschicht eine Membran aus gerecktem, mikroporösem PTFE aufweist.

20. Schuhwerk mit mindestens einer Fußschutzvorrichtung, dadurch gekennzeichnet, daß die Fußschutzvorrichtung eine Hartmaterialschale (25) aufweist, daß die Hartmaterialschale (25) auf mindestens ihrer Innenseite mit einem Polsterteil (27) aus geschlossenporigem Schaumstoff versehen ist und daß sowohl die Hartmaterialschale (25) als auch das Polsterteil (27) quer zu ihrer Längserstreckungsrichtung gelocht sind.

21. Schuhwerk nach Anspruch 20, dadurch gekennzeichnet, daß das Polsterteil (27) auf seiner Innenseite mit einem wasserdampfdurchlässigen Futter (31) versehen ist.

22. Schuhwerk nach Anspruch 20 oder 21, dadurch gekennzeichnet, daß die Fußschutzvorrichtung innerhalb eines wasserdampfdurchlässigen sowie winddichten und/oder wasserdichten Schuhaußenmaterials (35) angeordnet oder auf der Innenseite mit einem wasserdichten, wasserdampfdurchlässigen Material versehen ist.

23. Schuhwerk nach Anspruch 22, dadurch gekennzeichnet, daß das Schuhaußenmaterial (35) bzw. Material eine wasserdampfdurchlässige sowie winddichte und/oder wasserdichte Funktionsschicht aufweist.

24. Schuhwerk nach Anspruch 23, dadurch gekennzeichnet, daß die Funktionsschicht Teil eines das Schuhaußenmaterial (35) bildenden Laminats ist.

25. Schuhwerk nach einem der Ansprüche 22 bis 24, dadurch gekennzeichnet, daß die Funktionsschicht eine Membran aus gerecktem, mikroporösem PTFE aufweist.

26. Körperschienungsvorrichtung mit einer steifen Schienungsschale, dadurch gekennzeichnet, daß die Schienungsschale (51, 53) eine Hartmaterialschale (25) aufweist, daß die Innenseite der Hartmaterialschale (25) mit einem Polsterteil (27) aus geschlossenporigem Schaumstoff versehen ist und daß sowohl die Hartmaterialschale (25) als auch das Polsterteil (27) quer zu ihrer Längserstreckungsrichtung gelocht sind.

27. Körperschienungsvorrichtung nach Anspruch 26, dadurch gekennzeichnet, daß das Polsterteil (27) auf seiner Innenseite mit einem wasserdampfdurchlässigen Futter versehen ist.

28. Körperschienungsvorrichtung nach Anspruch 26 oder 27, dadurch gekennzeichnet, daß auf der Außenseite oder Innenseite der Hartmaterialschale (25) ein wasserdampfdurchlässiges sowie winddichtes und/oder wasserdichtes Material (35) angeordnet ist.

29. Köperschienungsvorrichtung nach Anspruch 28, dadurch gekennzeichnet, daß das Material eine wasserdampfdurchlässige sowie winddichte und/oder wasserdichte Funktionsschicht aufweist, vorzugsweise als Teil eines das Material (35) bildenden Laminats.

30. Körperschienungsvorrichtung nach Anspruch 28 oder 29, dadurch gekennzeichnet, daß die Funktionsschicht eine Membran aus gerecktem, mikroporösem PTFE aufweist.

FIG. 1

Aussen————————————Innen

FIG. 3

Aussen————————————Innen

FIG. 2

EP 0 490 137 A2

FIG. 4

FIG. 5

FIG. 6